(19) Europäisches Patentamt
European Patent Office
Office européen des brevets

(11) Publication number: **0 122 109 A2**

(12) # EUROPEAN PATENT APPLICATION

(21) Application number: **84302276.5**

(22) Date of filing: **03.04.84**

(51) Int. Cl.³: **C 07 D 213/73**, C 07 D 209/40, C 07 C 87/50, C 07 C 85/12 // C07D213/64, C07D213/61

(30) Priority: **07.04.83 GB 8309481**
**23.07.83 GB 8319876**

(43) Date of publication of application: **17.10.84**
**Bulletin 84/42**

(84) Designated Contracting States: **AT BE CH DE FR GB IT LI LU NL SE**

(71) Applicant: **SMITH KLINE & FRENCH LABORATORIES LIMITED, Mundells, Welwyn Garden City Hertfordshire, AL7 1EY (GB)**

(72) Inventor: **Adger, Brian Michael, 8 Tonbridge Road, Hildenborough Nr. Tonbridge Kent TN9 2Ng (GB)**
Inventor: **Wilczynska, Maria Aleksandra, 16 Moorlands, Welwyn Garden City Hertfordshire AL7 4PP (GB)**

(74) Representative: **Johnston, Walter Paul et al, Patent Department SMITH KLINE & FRENCH LABORATORIES LTD Mundells, Welwyn Garden City Hertfordshire AL7 1EY (GB)**

(54) **Preparation of cyano- or aminoalkyl substituted aromatic amines.**

(57) The present invention provides a process for preparing compounds of formula (5):

$$NH_2\text{-}AX\text{-}CH_2NH_2$$

(5)

which are useful as intermediates in the preparation of compounds which are histamine $H_1$-antagonists. In formula (5)

A is a 5-membered heterocyclic aromatic group containing up to three hetero-atoms of which no more than one can be oxygen or sulphur and from one to three can be nitrogen; a 6-membered monocyclic aromatic group optionally containing up to three nitrogen hetero-atoms or a 9- or 10-membered bicyclic aromatic group optionally containing up to four hetero-atoms, of which no more than one can be oxygen or sulphur and from one to four can be nitrogen, the group A being optionally substituted with one or more groups which are inert under the reaction conditions;

X is a $C_{1\text{-}5}$ alkylene group or a $C_{2\text{-}5}$ alkenyle group; which comprises (a) reacting a compound of formula (6):

$$NO_2\text{-}AX\text{-}CN$$

(6)

where A and X are as defined with reference to formula (5) with hydrazine in the presence of a transition metal catalyst to form a compound of formula (7):

$$NH_2\text{-}AX\text{-}CN$$

(7)

where A and X are as defined with reference to formula (5) and thereafter (b) reacting the compound of formula (7) so obtained with more hydrazine and Raney nickel.

EP 0 122 109 A2

TITLE MODIFIED
see front page

0122109



CHEMICAL PROCESS

This invention relates to a process for preparing cyanoalkyl substituted aromatic amines and for preparing aminoalkyl substituted aromatic amines.

European Patent Specification No 0068833 discloses and claims compounds of formula (1):-

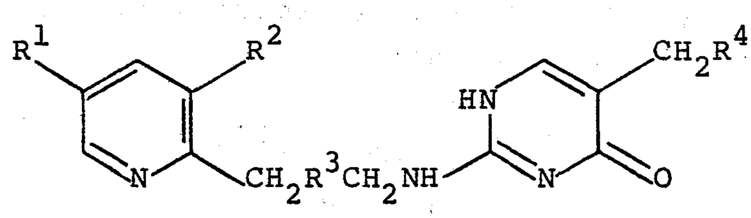

(1)

where $R^1$ is halogen, $R^2$ is $C_{1-4}$ alkyl, $R^3$ is $C_{1-3}$ alkylene and $R^4$ represents certain particular substituted or unsubstituted pyridine groups. These compounds are useful as histamine $H_1$-antagonists.

Key intermediates in the preparation of compounds of formula (1) are compounds of formula (2):-

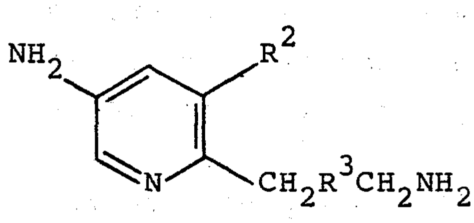

(2)

where $R^2$ and $R^3$ are as defined with reference to formula (1).

The compounds of formula (2) are prepared by hydrogenating a compound of formula (3):-

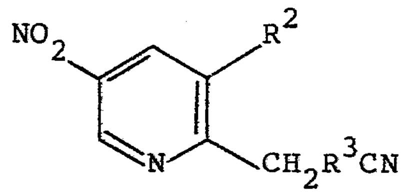

(3)

where $R^2$ and $R^3$ are as defined with reference to formula (1) and thereafter reducing the compound of formula (4) so obtained:-

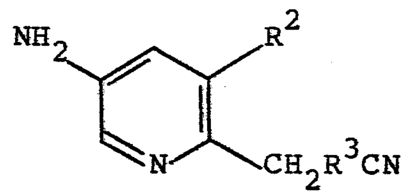

(4)

where $R^2$ and $R^3$ are as defined with reference to formula (1) with lithium aluminium hydride.

A process has now been invented in which the compounds of formula (2) can be prepared under mild conditions from the compounds of formula (3) and which allows compounds of formula (2) to be obtained in relatively high yield and relatively free of impurities.

It will be appreciated that this process is of rather broader application than simply for the preparation of compounds of formula (2).

Accordingly the present invention provides a process for preparing compounds of formula (5):-

$$NH_2\text{-}AX\text{-}CH_2NH_2$$

(5)

where A is a 5-membered heterocyclic aromatic group containing up to three hetero-atoms of which no more than one can be oxygen or sulphur and from one to three can be nitrogen; a 6-membered monocyclic aromatic group optionally containing up to three nitrogen hetero-atoms or a 9- or 10-membered bicyclic aromatic group optionally containing up to four hetero-atoms, of which no more than one can be oxygen or sulphur and from one to four can be nitrogen, the group A being optionally substituted with one or more groups which are inert under the reaction conditions;

X is a $C_{1-5}$ alkylene group or a $C_{2-5}$ alkenylene group; which comprises (a) reacting a compound of formula (6):-

$$NO_2\text{-AX-CN}$$
(6)

where A and X are as defined with reference to formula (5) with hydrazine in the presence of a transition metal catalyst to form a compound of formula (7):-

$$NH_2\text{-AX-CN}$$
(7)

where A and X are as defined with reference to formula (5) and thereafter (b) reacting the compound of formula (7) so obtained with more hydrazine and Raney nickel.

It will be appreciated that a group which is inert under the reaction conditions is one which is not reduced during the reaction or does not interfere with the reduction step. Examples of such groups are $C_{1-6}$ alkyl, $C_{1-6}$ alkoxy, halogen, amino or hydroxy groups.

Examples of 5-membered monocyclic aromatic groups containing one hetero atom are pyrrolyl, thienyl and furanyl. Examples of 5-membered aromatic groups containing two hetero-atoms are pyrazolyl, imidazolyl, thiazolyl, isothiazolyl, oxazolyl and isoxazolyl.

Examples of 5-membered monocyclic aromatic groups containing three hetero atoms are furazanyl, oxadiazolyl and thiadiazolyl.

Examples of 6-membered monocyclic aromatic groups optionally containing up to 3 nitrogen hetero atoms are phenyl, pyridyl, pyrazinyl, pyrimidinyl and pyridazinyl.

Examples of 9-membered bicyclic groups optionally containing up to 4 hetero atoms are, indinyl, indolyl, indazolyl, indolizinyl and purinyl.

Examples of 10-membered bicyclic groups optionally containing up to 4 hetero atoms are naphthyl, quinolyl, isoquinolyl, phthalazinyl, naphthyridinyl, quinoxalinyl, quinazolinyl, cinnolinyl and pteridinyl.

Where A in the compound of formula (6) is a 5-membered ring preferably it is imidazolyl, and in particular $NO_2$-A- is 5-nitro-4-imidazolyl.

Where A in the compound of formula (6) is a 6-membered monocyclic group it is in particular phenyl or pyridyl, and it is optionally substituted with a $C_{1-6}$ alkyl, $C_{1-6}$ alkoxy or halogen.

Thus in particular $NO_2$-A- is 2-nitrophenyl, 3-nitro-2-pyridyl, 5-nitro-2-pyridyl, 3-methyl-5-nitro-2-pyridyl, 5-methyl-3-nitro-2-pyridyl or 5-bromo-3-nitro-2-pyridyl. Preferably $NO_2$-A- is 3-methyl-5-nitro-2-pyridyl or 5-bromo-3-nitro-2-pyridyl.

Where A in the compound of formula (6) is a 9-membered bicyclic group, in particular it is 5-nitro-3-indolyl.

When X is a $C_{1-5}$ alkylene group it can represent for example ethane-1,2-diyl, propane-1,3-diyl, butane-1,4-diyl or pentane-1,5-diyl. In particular it is propane-1,3-diyl. When X is a $C_{2-5}$ alkenylene group, it can represent for example ethylene-1,2-diyl or prop-1-ene-1,3-diyl. In particular it is ethylene-1,2-diyl.

Preferably the first step of the reaction is carried out under mild conditions. That is, either a mild hydrogenation catalyst is employed at from low to slightly elevated temperature, or a more powerful catalyst is employed at from low temperatures to moderate.

An example of a mild catalyst for the first step of the reaction is palladium on an inert support (in particular palladium on charcoal). With a mild catalyst the reaction can be carried out at a temperature of from 5° to 70°C. In practice it is usually between room temperature (ca 20°C) and 55°C.

An example of a more powerful catalyst is Raney nickel. Although this catalyst can be used at a temperature of 70°C, preferably the reaction temperature is kept at or below 25°C, for example from 10° to 15°C to room temperature.

Preferably the catalyst is palladium on charcoal.

Step (b) is carried out using Raney nickel. The reaction can be carried out at from low to elevated temperatures, for example from 5°C to room temperature or higher, for example at temperatures in excess of 50°C and in particular at about 70°C.

Low temperatures are preferable in step (a) and step (b) where the group A is substituted with a group, for example halogen, which tends to be replaced by hydrogen under more forcing conditions.

After step (a) has been carried out, the compound of formula (7) can be recovered by removing the catalyst (e.g. by filtration) and evaporating the solvent. Step (b) can then be carried out by redissolving the compound of formula (7) so obtained in the same or a different solvent and reacting with Raney nickel and more hydrazine.

Preferably the reaction is carried out as a concerted process, that is by reacting the compound of formula (6) with sufficient hydrazine and a catalyst to form a compound of formula (7) *in situ*, where the catalyst for step (a) is not Raney nickel, removing the catalyst (e.g. by filtration) and then adding Raney nickel and sufficient hydrazine to convert the compound of formula (7) into the corresponding compound of formula (5).

The reaction of step (a) or step (b) can be carried out in the presence of a solvent the choice of which is not critical to the success of the reaction provided that it is substantially inert to the reagents and product. Examples of solvents for use in this process include $C_{1-6}$ alkanols in particular methanol and ethanol.

The time for which the reaction in step (a) or step (b) is allowed to proceed depends upon the nature of

reagents, the temperature at which it is carried out and in step (a), the catalyst. The progress of the reaction can be monitored by standard techniques for example thin layer chromatography, and when the reaction has finished, the product can be isolated by standard techniques, for example removing the catalyst by filtration and evaporating the solvent.

The following Examples illustrate the invention.

EXAMPLES

Example 1

(a) Palladium on charcoal (1.0g) was suspended in a solution of 4-(3-methyl-5-nitro-2-pyridyl) butyronitrile (9.6g) in methanol (100ml) and the mixture was heated to 35°C with stirring under a blanket of nitrogen. Aqueous hydrazine hydrate (64% w/v; 4.0ml) was added dropwise to the stirred suspension and the temperature of the reaction mixture was allowed to rise to 52°C. The temperature was maintained at 50°C until the reaction was shown by thin layer chromatography, to be complete (ca 1 hr.). The palladium on charcoal was removed by filtration. Removal of the solvent at this stage yields 4-(5-amino-3-methyl-2-pyridyl)butyronitrile. In this Example the filtrate was used directly in the next step.

(b) Moist Raney nickel (2 g) was added to the filtrate from Example 1(a). The mixture so obtained was heated to 50°C and aqueous hydrazine hydrate (64% w/v; 37 ml) was added dropwise with stirring maintaining the temperature at between 48-54°C. The temperature of the reaction mixture was maintained in this range and the progress of the reaction was followed by thin layer chromatography. When it was shown to be complete, the catalyst was removed by filtration and the solvent was evaporated to yield 4-(5-amino-3-methyl-2-pyridyl)-butylamine (6.87g; 82%).

Example 2

(a) A mixture of concentrated sulphuric acid (35 ml) and nitric acid (35 ml) was added dropwise with stirring to a chilled (5°C) solution of 2-amino-5-bromopyridine (50.3 g) in concentrated sulphuric acid (240 ml)

maintaining the temperature of the reaction mixture at 5°-6°C throughout the addition. When the addition was complete, the reaction mixture was stirred for a further 1.0 hr. at 5°-8°C and then warmed to 30°C and allowed to stand for ca 18 hr.

Further concentrated nitric acid (35 ml) was added portionwise to the reaction mixture with stirring while maintaining the temperature at 30°-40°C. A portion (50 ml) of the solution was poured into hot (ca 70°C) water (100 ml) with rapid stirring and this mixture was heated to 120°C. Gas evolved. When the evolution of gas ceased further portions (75 ml) of the reaction mixture were added maintaining the temperature at 120°C. When the additions were completed, the solution obtained was poured into ice (1 kg) and chilled in a salt/ice bath. Fine orange crystals formed which were removed by filtration and recrystallised from dimethylformamide/water to give 2-hydroxy-3-nitro-5-bromopyridine (23.5 g) m.p. 240°-243°C.

(b) A solution of 2-hydroxy-3-nitro-5-bromopyridine (23.4 g) in phosphoryl chloride (16 ml) was heated under reflux for 2.5 hr. The reaction mixture was poured into ice/water and a brown solid was produced which was removed by filtration. The solid was dissolved in chloroform, dried ($MgSO_4$) and decolourised by heating with charcoal for 30 min. The solvent was evaporated from the decolourised solution to yield a yellow solid (24.0 g) which was recrystallised from ether/petroleum ether (40°-60°C) to yield 2-chloro-3-nitro-5-bromopyridine (19.4 g) m.p. 66°-68°C.

(c) A solution of 2-(2-cyanoethyl)malonic acid diethyl ester (24.2 g) in tetrahydrofuran (15 ml) was added to a suspension of sodium hydride (2.45 g) in

tetrahydrofuran (30 ml) at 20°C under nitrogen. To this was added 2-chloro-3-nitro-5-bromopyridine (22 g) and the mixture so obtained was heated to 93°-95°C. A small amount of tetrahydrofuran was allowed to distil off. The mixture was heated under reflux for 2.5 hr. The reaction mixture was poured into water and neutralised to pH 7 with concentrated hydrochloric acid. The aqueous phase was extracted with chloroform, dried ($MgSO_4$) decolourised with charcoal and filtered through a silica column. The chloroform eluant was evaporated to yield an oil which slowly crystallised. The crystals were washed in petroleum ether (40°-60°C) and dried to yield 4-(5-bromo-3-nitropyrid-2-yl)-4,4-bis(carbethoxy)butyronitrile (28 g) m.p. 58°-62°C.

(d) Aqueous sodium hydroxide solution (1M, 263.6 ml) was added under nitrogen to a solution of 4-(5-bromo-3-nitropyrid-2-yl)-4,4-bis(carbethoxy)butyronitrile (21.8 g) in methanol (635 ml). The mixture so obtained was stirred for 18 hr. The mixture was acidified to pH 1.5 by addition of concentrated hydrochloric acid and heated at 50°C for 4.75 hr. The solution was neutralised to pH 7 with sodium hydroxide solution and the methanol removed by distillation. The aqueous solution remaining was extracted with chloroform to give an oil (11.2 g) which was chromatographed on a silica column with chloroform to give 4-[5-bromo-3-nitro-2-pyridyl] butyronitrile (9.6 g) as a yellow solid m.p. 73°-76°C.

(e) Raney nickel moist with ethanol (34 g) was added to a suspension of finely divided 4-[5-bromo-3-nitro-2-pyridyl] butyronitrile (8.4 g) in ethanol (350 ml) under nitrogen. The mixture was cooled (10°C) and a solution of hydrazine hydrate (2.34 ml) in ethanol (10 ml) was added maintaining the reaction temperature between 12°-15°C. The reaction mixture was allowed to warm to

room temperature with constant stirring and hydrazine hydrate (15.5 ml) was added in portions (2.3 ml) in ethanol (3 ml) at regular intervals over 46 hr. Before each addition the reaction mixture was cooled to 15°C. After 23 hr. more Raney nickel (6 g) was added. The reaction was stopped after 47 hr. The catalyst was removed by filtering the reaction mixture through a pad of diatomaceous earth. Evaporation of the solvent yielded an oil (7.9 g) which was chromatographed on a silica column eluting with ethyl acetate/ethanol/0.880 ammonia 15:10:2 to give 4-[3-amino-5-bromo-2-pyridyl] butylamine (4.0 g) as an oil.

Example 3

Substitution of 4-(3-nitro-2-pyridyl)butyronitrile (8.9 g) for 4-(3-methyl-5-nitro-2-pyridyl)butyronitrile in the process described in Example 1 yields 4-(3-amino-2-pyridyl)butylamine.

Example 4

Substitution of (2-nitrophenyl)acetonitrile (7.6 g) for 4-(3-methyl-5-nitro-2-pyridyl)butyronitrile in the process described in Example 1 yields 2-(2-aminophenyl)-ethylamine.

Example 5

Substitution of 2-(5-nitro-3-indolyl)proprionitrile for 4-(3-methyl-5-nitro-2-pyridyl)butyronitrile in the process described in Example 1 yields 2-(5-amino-3-indolyl)propylamine.

CLAIMS

1. A process for preparing a compound of formula (5):-

$$NH_2\text{-}AX\text{-}CH_2NH_2$$
(5)

where A is a 5-membered heterocyclic aromatic group containing up to three hetero-atoms of which no more than one can be oxygen or sulphur and from one to three can be nitrogen; a 6-membered monocyclic aromatic group optionally containing up to three nitrogen hetero-atoms or a 9- or 10-membered bicyclic aromatic group optionally containing up to four hetero-atoms, of which no more than one can be oxygen or sulphur and from one to four can be nitrogen, the group A being optionally substituted with one or more groups which are inert under the reaction conditions;

X is a $C_{1-5}$ alkylene group or a $C_{2-5}$ alkenylene group; which comprises (a) reacting a compound of formula (6):-

$$NO_2\text{-}AX\text{-}CN$$
(6)

where A and X are as defined with reference to formula (5) with hydrazine in the presence of a transition metal catalyst to form a compound of formula (7):-

$$NH_2\text{-}AX\text{-}CN$$
(7)

where A and X are as defined with reference to formula (5) and thereafter (b) reacting the compound of formula (7) so obtained with more hydrazine and Raney nickel.

2. A process as claimed in claim 1 where $NO_2$-A- is 2-nitrophenyl, 3-nitro-2-pyridyl, 5-nitro-2-pyridyl, 3-methyl-5-nitro-2-pyridyl, 5-methyl-3-nitro-2-pyridyl, 5-bromo-3-nitro-2-pyridyl or 5-nitro-3-indolyl.

3. A process as claimed in claim 2 where $NO_2$-A- is 3-methyl-5-nitro-2-pyridyl or 5-bromo-3-nitro-2-pyridyl.

4. A process as claimed in any one of claims 1 to 3 where X is propane-1,3-diyl.

5. A process as claimed in any one of claims 1 to 4 where the catalyst for step (a) is palladium on charcoal.

6. A process as claimed in any one of claims 1 to 5 in which the compound of formula (7) is formed <u>in situ</u> and, where the catalyst is other than Raney nickel, it is replaced with Raney nickel and reacted with a further quantity of hydrazine sufficient to produce the compound of formula (5).